(19) Europäisches Patentamt
European Patent Office
Office européen des brevets

(11) EP 4 790 043 A1

(12) **EUROPEAN PATENT APPLICATION**
published in accordance with Art. 153(4) EPC

(43) Date of publication:
**12.08.2026 Bulletin 2026/33**

(21) Application number: **24874568.9**

(22) Date of filing: **30.09.2024**

(51) International Patent Classification (IPC):
***C07C 1/22*** (2006.01) ***B01J 23/42*** (2006.01)
***B01J 23/44*** (2006.01) ***B01J 23/46*** (2006.01)
***B01J 29/18*** (2006.01) ***B01J 29/40*** (2006.01)
***B01J 29/44*** (2006.01) ***B01J 29/74*** (2006.01)
***C07B 61/00*** (2006.01) ***C07C 1/24*** (2006.01)
***C07C 9/15*** (2006.01) ***C07C 9/16*** (2006.01)
***C07C 9/22*** (2006.01)

(52) Cooperative Patent Classification (CPC):
**B01J 23/42; B01J 23/44; B01J 23/46; B01J 29/18; B01J 29/40; B01J 29/44; B01J 29/74; C07B 61/00; C07C 1/22; C07C 1/24; C07C 9/15; C07C 9/16; C07C 9/22**

(86) International application number:
**PCT/JP2024/034888**

(87) International publication number:
**WO 2025/074966 (10.04.2025 Gazette 2025/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.10.2023 JP 2023174317**
**18.09.2024 JP 2024161119**

(71) Applicant: **New Japan Chemical Co., Ltd.**
**Kyoto-shi, Kyoto 612-8224 (JP)**

(72) Inventor: **TAKAI, Hiromu**
**Soraku-gun, Kyoto 619-0237 (JP)**

(74) Representative: **Cabinet Beau de Loménie**
**103, rue de Grenelle / CS 90800**
**75340 Paris Cedex 07 (FR)**

(54) **METHOD FOR PRODUCING ALKANE**

(57) Provided is a method for producing an alkane that enables reactions to proceed at a lower temperature and a lower pressure, enables suppression of carbon loss from alcohols, and can achieve excellent conversion rate and selectivity.

The method for producing an alkane from a C8-C24 alcohol, the method including a step of reacting the C8-C24 alcohol with hydrogen under conditions including a reaction temperature of 160°C or higher and a hydrogen pressure of 3 MPa (gauge) or lower in the presence of a zeolite catalyst and a noble metal of Groups 8 to 10 of the periodic table, the zeolite catalyst being of type H-beta or type H-ZSM-5 and having a molar ratio ($SiO_2/Al_2O_3$) of silica ($SiO_2$) to alumina ($Al_2O_3$) of 20 to 35.



Processed by Luminess, 75001 PARIS (FR)

## Description

TECHNICAL FIELD

The present invention relates to methods for producing alkanes, and alkanes.

BACKGROUND ART

Methods for producing alkanes by using alcohols commonly found in nature as raw materials have been studied. Alkanes are chemically and thermally stable substances and are widely used in fields of catalysts for chemical reactions, coatings, or cosmetics, and the like.
Currently, alkanes used are mostly derived from petroleum and beneficially available at low cost and in large quantities.
However, use of petroleum-derived alkanes is considered to contribute to increase in the concentration of carbon dioxide on the earth. Therefore, there is a need to produce alkanes by using raw materials derived from biomass that are mainly made from plants.
Widely used plant-derived raw materials include oils and fats such as coconut oils, palm oils, castor oils, sunflower oils, and rapeseed oils. Using these raw materials, C8-C24 higher alcohols are produced around the world. Therefore, there is a need to produce alkanes by using the C8-C24 higher alcohols as raw materials.
Patent Literature 1, for example, discloses a method for producing linear saturated alkanes from primary alcohols whose carbon chain has one carbon atom more than the alkane, by reductive dehydroxymethylation of the primary alcohols in the presence of hydrogen and a catalyst at a temperature of 100°C to 300°C and a pressure of 1 to 250 bar, while removing water formed during the reaction, wherein C8-C24 fatty alcohols are used as primary alcohols.

CITATION LIST

- Patent Literature

Patent Literature 1: JP 2009-519264 T

SUMMARY OF INVENTION

- Technical Problem

In the method of Patent Literature 1, a reaction temperature of 220°C to 260°C and a reaction pressure of 10 to 50 bar are indicated as the most preferable ranges. A method for obtaining alkanes at a lower reaction temperature and a lower reaction pressure was required.
In the method of Patent Literature 1, alkanes with one fewer carbon atom than the primary alcohol are produced. Therefore, methane is also generated during the production of alkanes and operations such as methane recovery were required.
The present invention provides a method for producing an alkane that enables reactions to proceed at a lower temperature and a lower pressure, that enables suppression of carbon loss from alcohols, and that achieves excellent conversion rate and selectivity, than the method described in Patent Literature 1.

- Solution to Problem

From intense studies, the present inventors have found that reacting an alcohol with hydrogen by using a specific zeolite catalyst and a noble metal enables production of alkanes at a lower reaction temperature and a lower reaction pressure than conventional methods without carbon loss from the alcohol, and further achieves excellent conversion rate and selectivity, leading to the completion of the present invention.
Specifically, the present invention relates to a method for producing an alkane from a C8-C24 alcohol, the method including a step of reacting the C8-C24 alcohol with hydrogen under conditions including a reaction temperature of 160°C or higher and a hydrogen pressure of 3 MPa (gauge) or lower in the presence of a zeolite catalyst and a noble metal of Groups 8 to 10 of the periodic table, the zeolite catalyst being of type H-beta or type H-ZSM-5 and having a molar ratio ($SiO_2/Al_2O_3$) of silica ($SiO_2$) to alumina ($Al_2O_3$) of 20 to 35.
In the method for producing an alkane of the present invention, the step of reacting the C8-C24 alcohol with hydrogen is preferably performed by using the zeolite catalyst in an amount of 1 to 20% by mass relative to the mass of the C8-C24 alcohol.

In the method for producing an alkane of the present invention, the step of reacting the C8-C24 alcohol with hydrogen is preferably performed by using the noble metal of Groups 8 to 10 of the periodic table in an amount of 0.1 to 1% by mass relative to the mass of the C8-C24 alcohol.
The reaction temperature is preferably 180°C or higher.
The hydrogen pressure is preferably 1.0 MPa (gauge) or lower.
The hydrogen pressure is preferably 0 MPa (gauge) or higher.
The step of reacting the C8-C24 alcohol with hydrogen preferably has a reaction time of two to eight hours.
The zeolite catalyst preferably has a molar ratio ($SiO_2/Al_2O_3$) of silica ($SiO_2$) to alumina ($Al_2O_3$) of 24 to 30.
The noble metal of Groups 8 to 10 of the periodic table is preferably at least one selected from the group consisting of palladium, ruthenium, platinum, and rhodium.
The present invention also relates to an alkane having a natural origin index of 100% and a branched alkane content of 5% by mass or more and 70% by mass or less of the total alkane.

- Advantageous Effects of Invention

The present invention provides a method for producing an alkane that enables reactions to proceed at a lower temperature and a lower pressure, that enables suppression of carbon loss from alcohols, and that achieves excellent conversion rate and selectivity.

## DESCRIPTION OF EMBODIMENTS

The method for producing an alkane of the present invention relates to a method for producing an alkane from a C8-C24 alcohol, the method including a step of reacting the C8-C24 alcohol with hydrogen under conditions including a reaction temperature of 160°C or higher and a hydrogen pressure of 3 MPa (gauge) or lower in the presence of a zeolite catalyst and a noble metal of Groups 8 to 10 of the periodic table, the zeolite catalyst being of type H-beta or type H-ZSM-5 and having a molar ratio ($SiO_2/Al_2O_3$) of silica ($SiO_2$) to alumina ($Al_2O_3$) of 20 to 35.
Each constituent of the method for producing an alkane of the present invention will be described in detail below.

<Step of reacting C8-C24 alcohol with hydrogen>

The method for producing an alkane of the present invention includes the step of reacting the C8-C24 alcohol with hydrogen under conditions including a reaction temperature of 160°C or higher and a hydrogen pressure of 3 MPa (gauge) or lower in the presence of a zeolite catalyst and a noble metal of Groups 8 to 10 of the periodic table.

(Reaction temperature)

In the step of reacting the C8-C24 alcohol with hydrogen, the reaction temperature is 160°C or higher. When the reaction temperature is in this range, the reaction to obtain an alkane from the C8-C24 alcohol proceeds.
The reaction temperature is preferably 180°C or higher.
The upper limit thereof is not limited, and is, for example, 200°C.

(Hydrogen pressure)

In the step of reacting the C8-C24 alcohol with hydrogen, the hydrogen pressure is 3 MPa (gauge) or lower. When the hydrogen pressure is in this range, the reaction to obtain an alkane from the C8-C24 alcohol proceeds.
The hydrogen pressure is preferably 1.5 MPa (gauge) or lower, more preferably 1.0 MPa (gauge) or lower, still more preferably 0.5 MPa (gauge) or lower, particularly preferably 0.3 MPa (gauge) or lower.
The lower limit of the hydrogen pressure is preferably 0 MPa (gauge).
The reaction to obtain an alkane from the C8-C24 alcohol proceeds sufficiently even when the hydrogen pressure is at normal pressure. The reaction may proceed under reduced hydrogen pressure without impairing the effects.
Herein, a gauge pressure means the pressure excluding atmospheric pressure (defining atmospheric pressure as 0 MPa).

(Reaction time)

In the step of reacting the C8-C24 alcohol with hydrogen, the reaction time is preferably two to eight hours. When the reaction time is in this range, the reaction to obtain an alkane from the C8-C24 alcohol proceeds sufficiently.
From the perspective of the selectivity, the reaction time is more preferably three to four hours.

Herein, the reaction time means the period from reaching the above-mentioned reaction temperature to completing the reaction.

(Zeolite catalyst)

In the step of reacting the C8-C24 alcohol with hydrogen, a zeolite catalyst is used.
The zeolite catalyst is of type H-beta or type H-ZSM-5. Type H-beta refers to a zeolite having a beta-type (BEA-type) crystal structure and having hydrogen as a cation constituting the zeolite. Type H-ZSM-5 refers to a zeolite having a ZSM-5-type (MFI-type) crystal structure and having hydrogen as a cation constituting the zeolite.
The zeolite catalyst has a molar ratio ($SiO_2/Al_2O_3$) of silica ($SiO_2$) to alumina ($Al_2O_3$) of 20 to 35. When the molar ratio ($SiO_2/Al_2O_3$) is in this range, the reaction to obtain an alkane from the C8-C24 alcohol proceeds.
The molar ratio ($SiO_2/Al_2O_3$) is preferably 24 to 30, more preferably 24 to 28.
When the zeolite catalyst is of type H-beta, the molar ratio ($SiO_2/Al_2O_3$) is most preferably 28. Examples of commercially available products of such zeolite catalysts include HSZ-900 series, 931HOA-type (available from Tosoh Corporation).
When the zeolite catalyst is of type H-ZSM-5, the molar ratio ($SiO_2/Al_2O_3$) is most preferably 24 to 25. Examples of commercially available products of such zeolite catalysts include HSZ-800 series, 822HOA-type (available from Tosoh Corporation) and the like.
In the step of reacting the C8-C24 alcohol with hydrogen, from the perspective of suitably promoting the reaction to obtain an alkane from the C8-C24 alcohol, the zeolite catalyst is preferably used in an amount of 1 to 20% by mass relative to the mass of the C8-C24 alcohol.
When the zeolite catalyst is of type H-beta, the amount thereof is preferably 2% by mass or more relative to the mass of the C8-C24 alcohol.
When the zeolite catalyst is of type H-ZSM-5, the amount thereof is preferably 5% by mass or more relative to the mass of the C8-C24 alcohol.

(Noble metal of Groups 8 to 10 of periodic table)

In the step of reacting the C8-C24 alcohol with hydrogen, a noble metal of Groups 8 to 10 of the periodic table is used.
Examples of the noble metal of Groups 8 to 10 of the periodic table include ruthenium, osmium, cobalt, rhodium, iridium, nickel, palladium, platinum, and the like.
Among these, because of their safety and easy availability, the noble metal is preferably at least one selected from the group consisting of palladium, ruthenium, platinum, and rhodium.
More specific examples include palladium on carbon (Pd/C), palladium on alumina ($Pd/Al_2O_3$), ruthenium on carbon (Ru/C), ruthenium on alumina ($Ru/Al_2O_3$), rhodium on carbon (Rh/C), platinum on carbon (Pt/C), rhodium on carbon (Rh/C), and the like, with palladium on carbon (Pd/C) being particularly preferred.
In the step of reacting the C8-C24 alcohol with hydrogen, from the perspective of suitably promoting the reaction to obtain an alkane from the C8-C24 alcohol, the noble metal of Groups 8 to 10 of the periodic table is preferably used in an amount of 0.1 to 1% by mass relative to the mass of the C8-C24 alcohol.
The amount of the noble metal of Groups 8 to 10 of the periodic table is preferably 0.2% by mass or more, more preferably 0.3% by mass or more, relative to the mass of the C8-C24 alcohol.

(C8-C24 alcohol)

The C8-C24 alcohol is preferably a C8-C24 primary fatty alcohol.
Examples of the C8-C24 alcohol include 1-octanol, 1-nonanol, 1-decanol, 1-undecanol, 1-dodecanol, 1-tridecanol, 1-tetradecanol, 1-pentadecanol, 1-hexadecanol, 1-heptadecanol, 1-octadecanol, 1-nonadecanol, 1-eicosanol, 1-docosanol, and the like.
These alcohols used as raw materials may be used alone or in combination of two or more thereof.
In the method for producing an alkane of the present invention, an alkane having the same number of carbon atoms as the C8-C24 alcohol can be produced.

(Others)

In the step of reacting the C8-C24 alcohol with hydrogen, a solvent may be used, but it is preferable not to use a solvent. When a solvent is used, the solvent should have a boiling point equal to of higher than the reaction temperature for obtaining an alkane from the C8-C24 alcohol.

The step of reacting the C8-C24 alcohol with hydrogen may be performed in a closed system or a flow system. In either case, it is necessary to remove water produced by the reaction of the C8-C24 alcohol with hydrogen.

For example, in the closed system, purge operations are conducted as appropriate by opening and closing valves to remove water produced by the reaction of the C8-C24 alcohol with hydrogen.

Alternatively, in the flow system, using an open-system reactor, both water produced by the reaction of the C8-C24 alcohol with hydrogen and unreacted hydrogen are released from the system, while hydrogen is continuously supplied thereto.

Additionally, in the flow system, it is necessary to adjust the flow rate of hydrogen so as not to release alcohol used as a raw material from the system in the reaction to obtain an alkane from the C8-C24 alcohol.

Examples of apparatus used in the step of reacting the C8-C24 alcohol with hydrogen are not limited and well-known apparatus may be selected as appropriate.

After the step of reacting the C8-C24 alcohol with hydrogen, purification may be performed.

Examples of the purification include methods such as filtration of catalysts used in the step of reacting the C8-C24 alcohol with hydrogen, and purification by distilling the filtered crude product.

The method of purification can be any well-known one.

(Method of analysis)

The production of alkanes by the method for producing an alkane of the present invention can be confirmed by gas chromatography analysis (GC).

The linear alkane content and the branched alkane content can also be determined by the GC.

Specifically, the methods described in EXAMPLES herein may be used.

The method for producing an alkane of the present invention enables the production of alkane with a high conversion rate and high selectivity. Herein, "conversion rate" means the consumption of alcohol used as a raw material. The conversion rate can be expressed by the following formula.

Conversion rate (%) = 100 (%) - proportion of alcohol used as raw material that remained unreacted after reaction of C8-C24 alcohol with hydrogen (%)

When alcohol used as a raw material is completely consumed, the conversion rate is defined as 100%. The proportion of alcohol used as a raw material that remained unreacted after the reaction of C8-C24 alcohol with hydrogen (%) can be determined by the GC.

Herein, "selectivity" means the proportion of the target product in the formula for the conversion rate. The target product is an alkane without carbon loss from alcohols used as a raw material. The selectivity can be expressed by the following formula.

$$\text{Selectivity (\%)} = < (\text{proportion of target product (\%)}) / (\text{conversion rate}) > \times 100$$

In the method for producing an alkane of the present invention, the conversion rate is 40% or higher.

The conversion rate is preferably 50% or higher, more preferably 60% or higher, still more preferably 70% or higher, further preferably 80% or higher, particularly preferably 90% or higher, most preferably 100%.

In the method for producing an alkane of the present invention, the selectivity is 55% or higher.

The selectivity is preferably 60% or higher, more preferably 70% or higher, still more preferably 80% or higher, further preferably 90% or higher, particularly preferably 95% or higher, most preferably 100%.

(Alkane)

In the method for producing an alkane of the present invention, an alkane having a natural origin index of 100% is obtainable by using a biomass-derived raw material.

In other words, in the method for producing an alkane of the present invention, the alkane having a natural origin index of 100% and a branched alkane content of 5% by mass or more and 70% by mass or less of the total alkane is obtainable.

The present invention encompasses such an alkane obtained by the method for producing an alkane of the present invention.

Herein, the natural origin index refers to the value to be calculated in conformity with ISO 16128 and indicates the proportion of natural raw materials derived from plants, animals, algae, microorganisms such as bacteria and fungi, and minerals.

The alkane of the present invention has a natural origin index of 100% and therefore all raw materials thereof originate from natural sources.

Herein, the following items are disclosed.

The present disclosure (1) relates to a method for producing an alkane from a C8-C24 alcohol, the method including a step of reacting the C8-C24 alcohol with hydrogen under conditions including a reaction temperature of 160°C or higher and a hydrogen pressure of 3 MPa (gauge) or lower in the presence of a zeolite catalyst and a noble metal of Groups 8 to 10 of the periodic table, the zeolite catalyst being of type H-beta or type H-ZSM-5 and having a molar ratio ($SiO_2/Al_2O_3$) of silica ($SiO_2$) to alumina ($Al_2O_3$) of 20 to 35.

The present disclosure (2) relates to the method for producing an alkane according to disclosure (1), wherein the step of reacting the C8-C24 alcohol with hydrogen is performed by using the zeolite catalyst in an amount of 1 to 20% by mass relative to the mass of the C8-C24 alcohol.

The present disclosure (3) relates to the method for producing an alkane according to disclosure (1) or (2), wherein the step of reacting the C8-C24 alcohol with hydrogen is performed by using the noble metal of Groups 8 to 10 of the periodic table in an amount of 0.1 to 1% by mass relative to the mass of the C8-C24 alcohol.

The present disclosure (4) relates to the method for producing an alkane according to any of disclosures (1) to (3), wherein the reaction temperature is 180°C or higher.

The present disclosure (5) relates to the method for producing an alkane according to any of disclosures (1) to (4), wherein the hydrogen pressure is 1.0 MPa (gauge) or lower.

The present disclosure (6) relates to the method for producing an alkane according to any of disclosures (1) to (5), wherein the hydrogen pressure is 0 MPa (gauge) or higher.

The present disclosure (7) relates to the method for producing an alkane according to any of disclosures (1) to (6), wherein the step of reacting the C8-C24 alcohol with hydrogen has a reaction time of two to eight hours.

The present disclosure (8) relates to the method for producing an alkane according to any of disclosures (1) to (7), wherein the zeolite catalyst has a molar ratio ($SiO_2/Al_2O_3$) of silica ($SiO_2$) to alumina ($Al_2O_3$) of 24 to 30.

The present disclosure (9) relates to the method for producing an alkane according to any of disclosures (1) to (8), wherein the noble metal of Groups 8 to 10 of the periodic table is at least one selected from the group consisting of palladium, ruthenium, platinum, and rhodium.

The present disclosure (10) relates to an alkane having a natural origin index of 100% and a branched alkane content of 5% by mass or more and 70% by mass or less of the total alkane.

## EXAMPLES

The present invention is described in more detail below with reference to examples, but is not limited to these examples. For compounds not explicitly mentioned, reagents were used.

### (Zeolite catalyst)

- 822HOA (HSZ-800 series, 822HOA-type, available from Tosoh Corporation)
- 840HOA (HSZ-800 series, 840HOA-type, available from Tosoh Corporation)
- 620HOA (HSZ-600 series, 620HOA-type, available from Tosoh Corporation)
- 660HOA (HSZ-600 series, 660HOA-type, available from Tosoh Corporation)
- 320HOA (HSZ-300 series, 320HOA-type, available from Tosoh Corporation)
- 360HOA (HSZ-300 series, 360HOA-type, available from Tosoh Corporation)
- 931HOA (HSZ-900 series, 931HOA-type, available from Tosoh Corporation)
- 940HOA (HSZ-900 series, 940HOA-type, available from Tosoh Corporation)
- Zeolite catalyst (available from N.E. Chemcat Corporation)

Tables 1 to 4 show crystal forms and molar ratios ($SiO_2/Al_2O_3$) of silica ($SiO_2$) to alumina ($Al_2O_3$). The molar ratios ($SiO_2/Al_2O_3$) were taken from the catalog values.

H-MOR refers to a zeolite having a mordenite-type crystal structure and having hydrogen as a cation constituting the zeolite. H-Y refers to a zeolite having a Y-type crystal structure and having hydrogen as a cation constituting the zeolite.

Each usage (% by mass) shown in Tables 1 to 4 means the usage (% by mass) of the zeolite catalyst relative to the mass of the C8-C24 alcohol.

(Noble metal of Groups 8 to 10 of periodic table)

- Pd/C (palladium on carbon, available from N.E. Chemcat Corporation)
- Pd/$Al_2O_3$ (palladium on alumina, available from N.E. Chemcat Corporation)
- Ru/C (ruthenium on carbon, available from N.E. Chemcat Corporation)
- Pt/C (platinum on carbon, available from N.E. Chemcat Corporation)
- Rh/C (rhodium on carbon, available from N.E. Chemcat Corporation)

Each usage (% by mass) shown in Tables 1 to 4 means the usage (% by mass) of the noble metal of Groups 8 to 10 of the periodic table relative to the mass of the C8-C24 alcohol.

Moreover, each of the noble metals of Groups 8 to 10 of the periodic table has a 5% metal loading and is a dry product.

(C8-C24 alcohol)

- $C_8OH$ (1-octanol) (CONOL 10WS: plant-derived (biomass-derived raw material), available from New Japan Chemical Co., Ltd.)
- $C_{10}OH$ (1-decanol) (CONOL 1098: plant-derived, available from New Japan Chemical Co., Ltd.)
- $C_{12}OH$ (1-dodecanol) (CONOL 20P: plant-derived, available from New Japan Chemical Co., Ltd.)
- $C_{14}OH$ (1-tetradecanol) (CONOL 1495: plant-derived, available from New Japan Chemical Co., Ltd.)
- $C_{16}OH$ (1-hexadecanol) (CONOL 1695: plant-derived, available from New Japan Chemical Co., Ltd.)
- $C_{18}OH$ (1-octadecanol) (CONOL 30SS: plant-derived, available from New Japan Chemical Co., Ltd.)
- $C_{22}OH$ (1-docosanol) (available from Tokyo Chemical Industry Co., Ltd.)
- $C_{12}OH$ (75% by mass) - $C_{14}OH$ (25% by mass) (CONOL C1275: plant-derived, available from New Japan Chemical Co., Ltd.)

(Comparative Example 1)

A stirred pressure vessel was charged with 100g of 1-dodecanol (0.54 mol, available from New Japan Chemical Co., Ltd.) and 5g of type H-ZSM-5 (HSZ-800 series, 822HOA-type, available from Tosoh Corporation) together and heated to the reaction temperature of 180°C at a stirring rate of 1000 rpm. After reaching the reaction temperature of 180°C, the reaction vessel was purged every 20 minutes for 4 hours until the internal pressure was reduced to 0 MPa (gauge). Subsequently, the product was cooled, taken out, and filtered, thereby yielding 96g of reaction product.

(Comparative Examples 2 to 8, and 17)

Alkanes were produced in the same manner as in Comparative Example 1, except that catalyst systems used were changed as shown in Tables 1 to 2.

(Example 1)

A stirred pressure vessel was charged with 100g of 1-dodecanol (0.54 mol, available from New Japan Chemical Co., Ltd.), 5g of type H-ZSM-5 (HSZ-800 series, 822HOA-type, available from Tosoh Corporation), and 0.5g of 5% Pd/C (available from N.E. Chemcat Corporation, dry product) together and heated to the reaction temperature of 180°C at a stirring rate of 1000 rpm. For four hours after reaching the reaction temperature of 180°C, each time the internal pressure of the reaction vessel reached 0.02 MPa, the reaction vessel was purged through a discharge valve attached thereto until the internal pressure reached 0 MPa (gauge), and hydrogen was then supplied until the internal pressure reached 0.1 MPa (gauge). Subsequently, the product was cooled, taken out, and filtered, thereby yielding 90g of reaction product.

(Examples 2 to 43 and Comparative Examples 9 to 16)

Alkanes were produced in the same manner as in Example 1, except that reaction temperatures, hydrogen pressures, reaction times, catalyst systems used, and C8-C24 alcohols were changed as shown in Tables 1 to 4. The hydrogen pressures shown in Tables 1 to 4 are expressed as gauge pressure values.

(GC analysis)

In the GC analysis, the products obtained in each example and comparative example were subject to filtration to remove the catalyst, and the filtered crude products each were adjusted to a 1 mass% hexane solution to serve as measurement samples. GC measurement conditions are described below. The measurements were performed as described below, depending on the number of carbon atoms of the alcohol used as a raw material.

The conversion rate and selectivity were calculated by the methods described herein. The products having a conversion rate of 40% or higher and a selectivity of 55% or higher were defined as acceptable.

In addition, the linear alkane content and the branched alkane content were determined by the GC.

<Measurement conditions>

<GC Measurement conditions for C8-C10 analysis>

Model: Gas chromatograph GC-2014 (available from Shimadzu Corporation)
Detector: FID, 335°C
Column: DB-1 (30 m × ϕ0.25 mm × 0.25 μm)
Column temperature: 40°C
Injection temperature: 335°C
Carrier gas: helium (linear velocity: 40 cm/sec)
Injection volume: 1.0 μl (split ratio: 50)

<GC Measurement conditions for C12-C18 analysis>

Model: Gas chromatograph GC-2014 (available from Shimadzu Corporation)
Detector: FID, 335°C
Column: DB-1 (30 m × Φ0.25 mm × 0.25 μm)
Column temperature: 70°C
Injection temperature: 335°C
Carrier gas: helium (linear velocity: 40 cm/sec)
Injection volume: 1.0 μl (split ratio: 50)

<GC Measurement conditions for C20 or more analysis>

Model: Gas chromatograph GC-2014 (available from Shimadzu Corporation)
Detector: FID, 335°C
Column: DB-1 (30 m × Φ0.25 mm × 0.25 μm)
Column temperature: 100°C
Injection temperature: 335°C
Carrier gas: helium (linear velocity: 38 cm/sec)
Injection volume: 1.0 μl (split ratio: 50)

[Table 1]

| | Reaction temperature (°C) | Hydrogen pressure (Mpa) | Reaction time (h) | Zeolite catalyst | | | | Noble metal of Groups 8 to 10 of periodic table | | C8-C24 alcohol | Conversion rate (%) | Selectivity (%) |
|---|---|---|---|---|---|---|---|---|---|---|---|---|
| | | | | Type | Crystal form | Molar ratio ($SiO_2/Al_2O_3$) | Usage (% by mass) | Type | Usage (% by mass) | | | |
| Comparative Example 1 | 180 | Atmospheric pressure | 4 | 822HOA | H-ZSM-5 | 24 | 5 | - | 0 | $C_{12}OH$ | 29.6 | 15.6 |
| Comparative Example 2 | 180 | Atmospheric pressure | 4 | 840HOA | H-ZSM-5 | 40 | 5 | - | 0 | $C_{12}OH$ | 0.0 | - |
| Comparative Example 3 | 180 | Atmospheric pressure | 4 | 620HOA | H-MOR | 15 | 5 | - | 0 | $C_{12}OH$ | 0.0 | - |
| Comparative Example 4 | 180 | Atmospheric pressure | 4 | 660HOA | H-MOR | 30 | 5 | - | 0 | $C_{12}OH$ | 1.9 | 21.3 |
| Comparative Example 5 | 180 | Atmospheric pressure | 4 | 320HOA | H-Y | 5.5 | 5 | - | 0 | $C_{12}OH$ | 7.1 | 14.1 |
| Comparative Example 6 | 180 | Atmospheric pressure | 4 | 360HOA | H-Y | 15 | 5 | - | 0 | $C_{12}OH$ | 14.8 | 3.7 |
| Comparative Example 7 | 180 | Atmospheric pressure | 4 | 931HOA | H-Beta | 28 | 5 | - | 0 | $C_{12}OH$ | 77.7 | 7.6 |
| Comparative Example 8 | 180 | Atmospheric pressure | 4 | 940HOA | H-Beta | 40 | 5 | - | 0 | $C_{12}OH$ | 97.9 | 2.1 |
| Example 1 | 180 | 0.1 | 4 | 822HOA | H-ZSM-5 | 24 | 5 | Pd/C | 0.5 | $C_{12}OH$ | 71.8 | 78.7 |
| Example 2 | 180 | 0.1 | 4 | 822HOA | H-ZSM-5 | 24 | 5 | $Pd/Al_2O_3$ | 0.5 | $C_{12}OH$ | 58.2 | 81.6 |
| Example 3 | 180 | 0.1 | 4 | 822HOA | H-ZSM-5 | 24 | 5 | Ru/C | 0.5 | $C_{12}OH$ | 50.8 | 67.6 |
| Example 4 | 180 | 0.1 | 4 | 822HOA | H-ZSM-5 | 24 | 5 | Pt/C | 0.5 | $C_{12}OH$ | 50.9 | 76.1 |
| Example 5 | 180 | 0.1 | 4 | 822HOA | H-ZSM-5 | 24 | 5 | Rh/C | 0.5 | $C_{12}OH$ | 49.1 | 71.6 |

[Table 2]

| | Reaction temperature (°C) | Hydrogen pressure (Mpa) | Reaction time (h) | Zeolite catalyst | | | | Noble metal of Groups 8 to 10 of periodic table | | C8-C24 alcohol | Conversion rate (%) | Selectivity (%) |
|---|---|---|---|---|---|---|---|---|---|---|---|---|
| | | | | Type | Crystal form | Molar ratio ($SiO_2/Al_2O_3$) | Usage (% by mass) | Type | Usage (% by mass) | | | |
| Comparative Example 9 | 180 | 0.1 | 4 | 840HOA | H-ZSM-5 | 40 | 5 | Pd/C | 0.5 | $C_{12}OH$ | 3.8 | 25.6 |
| Comparative Example 10 | 180 | 0.1 | 4 | 620HOA | H-MOR | 15 | 5 | Pd/C | 0.5 | $C_{12}OH$ | 5.3 | 16.2 |
| Comparative Example 11 | 180 | 0.1 | 4 | 660HOA | H-MOR | 30 | 5 | Pd/C | 0.5 | $C_{12}OH$ | 4.4 | 10.0 |
| Comparative Example 12 | 180 | 0.1 | 4 | 320HOA | H-Y | 5.5 | 5 | Pd/C | 0.5 | $C_{12}OH$ | 8.8 | 48.8 |
| Comparative Example 13 | 180 | 0.1 | 4 | 360HOA | H-Y | 15 | 5 | Pd/C | 0.5 | $C_{12}OH$ | 14.9 | 15.6 |
| Example 6 | 180 | 0.1 | 4 | 931HOA | H-Beta | 28 | 5 | Pd/C | 0.5 | $C_{12}OH$ | 100.0 | 77.6 |
| Comparative Example 14 | 180 | 0.1 | 4 | 940HOA | H-Beta | 40 | 5 | Pd/C | 0.5 | $C_{12}OH$ | 97.9 | 20.8 |
| Example 7 | 180 | 0.1 | 4 | 822HOA | H-ZSM-5 | 24 | 10 | Pd/C | 0.5 | $C_{12}OH$ | 100.0 | 86.1 |
| Example 8 | 180 | 0.1 | 4 | 822HOA | H-ZSM-5 | 24 | 10 | Pd/C | 0.1 | $C_{12}OH$ | 99.7 | 83.8 |
| Example 9 | 180 | 0.1 | 4 | 822HOA | H-ZSM-5 | 24 | 10 | Pd/C | 0.3 | $C_{12}OH$ | 100.0 | 83.7 |
| Example 10 | 180 | 0.1 | 4 | 822HOA | H-ZSM-5 | 24 | 10 | Pd/C | 1 | $C_{12}OH$ | 100.0 | 85.0 |
| Comparative Example 15 | 180 | 0.1 | 4 | 822HOA | H-ZSM-5 | 24 | 10 | - | 0 | $C_{12}OH$ | 68.7 | 10.6 |
| Comparative Example 16 | 180 | 0.1 | 4 | - | - | - | 0 | Pt/C | 0.5 | $C_{12}OH$ | 0.0 | - |
| Comparative Example 17 | 180 | Atmospheric pressure | 4 | 822HOA | H-ZSM-5 | 24 | 10 | Pd/C | 0.5 | $C_{12}OH$ | 69.4 | 10.7 |

[Table 3]

| | Reaction temperature (°C) | Hydrogen pressure (Mpa) | Reaction time (h) | Zeolite catalyst | | | | Noble metal of Groups 8 to 10 of periodic table | | C8-C24 alcohol | Conversion rate (%) | Selectivity (%) |
|---|---|---|---|---|---|---|---|---|---|---|---|---|
| | | | | Type | Crystal form | Molar ratio ($SiO_2/Al_2O_3$) | Usage (% by mass) | Type | Usage (% by mass) | | | |
| Example 11 | 160 | 0.1 | 8 | 822HOA | H-ZSM-5 | 24 | 10 | Pd/C | 0.3 | $C_{12}OH$ | 43.5 | 78.5 |
| Example 12 | 170 | 0.1 | 4 | 822HOA | H-ZSM-5 | 24 | 10 | Pd/C | 0.3 | $C_{12}OH$ | 54.4 | 81.6 |
| Example 13 | 170 | 0.1 | 6 | 822HOA | H-ZSM-5 | 24 | 10 | Pd/C | 0.3 | $C_{12}OH$ | 79.1 | 81.3 |
| Example 14 | 180 | 0.1 | 2 | 822HOA | H-ZSM-5 | 24 | 10 | Pd/C | 0.3 | $C_{12}OH$ | 48.7 | 85.7 |
| Example 15 | 180 | 0.1 | 3 | 822HOA | H-ZSM-5 | 24 | 10 | Pd/C | 0.3 | $C_{12}OH$ | 70.0 | 85.2 |
| Example 16 | 180 | 0.1 | 4 | 822HOA | H-ZSM-5 | 24 | 10 | Pd/C | 0.3 | $C_{12}OH$ | 100.0 | 83.7 |
| Example 17 | 200 | 0.1 | 2 | 822HOA | H-ZSM-5 | 24 | 10 | Pd/C | 0.3 | $C_{12}OH$ | 100.0 | 64.9 |
| Example 18 | 200 | 0.1 | 3 | 822HOA | H-ZSM-5 | 24 | 10 | Pd/C | 0.3 | $C_{12}OH$ | 100.0 | 62.7 |
| Example 19 | 160 | 0.1 | 8 | 931HOA | H-Beta | 28 | 2 | Pd/C | 0.1 | $C_{12}OH$ | 60.0 | 59.6 |
| Example 20 | 170 | 0.1 | 4 | 931HOA | H-Beta | 28 | 2 | Pd/C | 0.1 | $C_{12}OH$ | 74.5 | 62.5 |
| Example 21 | 170 | 0.1 | 6 | 931HOA | H-Beta | 28 | 2 | Pd/C | 0.1 | $C_{12}OH$ | 100.0 | 69.0 |
| Example 22 | 180 | 0.1 | 2 | 931HOA | H-Beta | 28 | 2 | Pd/C | 0.1 | $C_{12}OH$ | 65.3 | 63.4 |

(continued)

| | Reaction temperature (°C) | Hydrogen pressure (Mpa) | Reaction time (h) | Zeolite catalyst | | | | Noble metal of Groups 8 to 10 of periodic table | | C8-C24 alcohol | Conversion rate (%) | Selectivity (%) |
|---|---|---|---|---|---|---|---|---|---|---|---|---|
| | | | | Type | Crystal form | Molar ratio ($SiO_2/Al_2O_3$) | Usage (% by mass) | Type | Usage (% by mass) | | | |
| Example 23 | 180 | 0.1 | 3 | 931HOA | H-Beta | 28 | 2 | Pd/C | 0.1 | $C_{12}OH$ | 95.4 | 64.6 |
| Example 24 | 180 | 0.1 | 4 | 931HOA | H-Beta | 28 | 2 | Pd/C | 0.1 | $C_{12}OH$ | 100.0 | 70.3 |
| Example 25 | 200 | 0.1 | 2 | 931HOA | H-Beta | 28 | 2 | Pd/C | 0.1 | $C_{12}OH$ | 100.0 | 56.9 |
| Example 26 | 200 | 0.1 | 3 | 931HOA | H-Beta | 28 | 2 | Pd/C | 0.1 | $C_{12}OH$ | 100.0 | 56.1 |

[Table 4]

| | Reaction temperature (°C) | Hydrogen pressure (Mpa) | Reaction time (h) | Zeolite catalyst | | | | Noble metal of Groups 8 to 10 of periodic table | | C8-C24 alcohol | Conversion rate (%) | Selectivity (%) |
|---|---|---|---|---|---|---|---|---|---|---|---|---|
| | | | | Type | Crystal form | Molar ratio ($SiO_2/Al_2O_3$) | Usage (% by mass) | Type | Usage (% by mass) | | | |
| Example 27 | 180 | 0 | 4 | 822HOA | H-ZSM-5 | 24 | 10 | Pd/C | 0.3 | $C_{12}OH$ | 100.0 | 86.3 |
| Example 28 | 180 | 0.1 | 4 | 822HOA | H-ZSM-5 | 24 | 10 | Pd/C | 0.3 | $C_{12}OH$ | 100.0 | 83.7 |
| Example 29 | 180 | 0.3 | 4 | 822HOA | H-ZSM-5 | 24 | 10 | Pd/C | 0.3 | $C_{12}OH$ | 100.0 | 86.3 |
| Example 30 | 180 | 0.5 | 4 | 822HOA | H-ZSM-5 | 24 | 10 | Pd/C | 0.3 | $C_{12}OH$ | 100.0 | 82.4 |
| Example 31 | 180 | 1.0 | 4 | 822HOA | H-ZSM-5 | 24 | 10 | Pd/C | 0.3 | $C_{12}OH$ | 91.5 | 84.1 |
| Example 32 | 180 | 1.5 | 4 | 822HOA | H-ZSM-5 | 24 | 10 | Pd/C | 0.3 | $C_{12}OH$ | 75.4 | 85.1 |
| Example 33 | 180 | 2.0 | 4 | 822HOA | H-ZSM-5 | 24 | 10 | Pd/C | 0.3 | $C_{12}OH$ | 80.3 | 82.1 |
| Example 34 | 180 | 3.0 | 4 | 822HOA | H-ZSM-5 | 24 | 10 | Pd/C | 0.3 | $C_{12}OH$ | 83.3 | 82.1 |
| Example 35 | 180 | 0.1 | 4 | 822HOA | H-ZSM-5 | 24 | 10 | Pd/C | 0.3 | $C_8OH$ | 95.2 | 72.1 |
| Example 36 | 180 | 0.1 | 4 | 822HOA | H-ZSM-5 | 24 | 10 | Pd/C | 0.3 | $C_{10}OH$ | 98.1 | 80.2 |
| Example 37 | 180 | 0.1 | 4 | 822HOA | H-ZSM-5 | 24 | 10 | Pd/C | 0.3 | $C_{14}OH$ | 100.0 | 85.3 |
| Example 38 | 180 | 0.1 | 4 | 822HOA | H-ZSM-5 | 24 | 10 | Pd/C | 0.3 | $C_{16}OH$ | 100.0 | 92.1 |

(continued)

| | Reaction temperature (°C) | Hydrogen pressure (Mpa) | Reaction time (h) | Zeolite catalyst | | | | Noble metal of Groups 8 to 10 of periodic table | | C8-C24 alcohol | Conversion rate (%) | Selectivity (%) |
|---|---|---|---|---|---|---|---|---|---|---|---|---|
| | | | | Type | Crystal form | Molar ratio ($SiO_2/Al_2O_3$) | Usage (% by mass) | Type | Usage (% by mass) | | | |
| Example 39 | 180 | 0.1 | 4 | 822HOA | H-ZSM-5 | 24 | 10 | Pd/C | 0.3 | $C_{18}OH$ | 100.0 | 88.2 |
| Example 40 | 180 | 0.1 | 4 | 822HOA | H-ZSM-5 | 24 | 10 | Pd/C | 0.3 | $C_{22}OH$ | 100.0 | 91.9 |
| Example 41 | 180 | 0.1 | 4 | 822HOA | H-ZSM-5 | 24 | 10 | Pd/C | 0.3 | $C_{12}OH$ (75% by mass) $C_{14}OH$ (25% by mass) | 100.0 | 84.0 |
| Example 42 | 180 | 0.1 | 4 | Zeolite catalyst | H-ZSM-5 | 25 | 10 | Pd/C | 0.3 | $C_{12}OH$ | 71.8 | 75.8 |
| Example 43 | 180 | 0 | 6 | 822HOA | H-ZSM-5 | 24 | 10 | Pd/C | 0.01 | $C_{12}OH$ | 100.0 | 81.5 |

The examples demonstrated that the method for producing an alkane of the present invention enabled reactions to proceed at a lower temperature (160°C to 200°C) and a lower pressure (hydrogen pressure: 3.0 MPa (gauge) or lower), enabled suppression of carbon loss from alcohols, and achieved excellent conversion rate and selectivity, than conventional methods for producing alkanes.

The alkanes produced by the method for producing an alkane of the present invention had a natural origin index of 100% because of the use of biomass-derived raw materials.

The alkane produced in Example 8 had a branched alkane content of 15% by mass and a linear alkane content of 85% by mass.

The alkane produced in Example 38 had a branched alkane content of 5% by mass and a linear alkane content of 92% by mass.

The alkane produced in Example 18 had a branched alkane content of 21% by mass and a linear alkane content of 63% by mass. Further distillation of these alkanes yielded alkanes having a branched alkane content of 66% by mass.

INDUSTRIAL APPLICABILITY

The method for producing an alkane of the present invention enables reactions to proceed at a lower temperature and a lower pressure, enables suppression of carbon loss from alcohols, and achieves excellent conversion rate and selectivity.

The alkane of the present invention can be used as raw material for cosmetics and biomass-derived products.

## Claims

**1.** A method for producing an alkane from a C8-C24 alcohol, the method comprising

a step of reacting the C8-C24 alcohol with hydrogen under conditions including a reaction temperature of 160°C or higher and a hydrogen pressure of 3 MPa (gauge) or lower in the presence of a zeolite catalyst and a noble metal of Groups 8 to 10 of the periodic table,
the zeolite catalyst being of type H-beta or type H-ZSM-5 and having a molar ratio ($SiO_2/Al_2O_3$) of silica ($SiO_2$) to alumina ($Al_2O_3$) of 20 to 35.

**2.** The method for producing an alkane according to claim 1,
wherein the step of reacting the C8-C24 alcohol with hydrogen is performed by using the zeolite catalyst in an amount of 1 to 20% by mass relative to the mass of the C8-C24 alcohol.

**3.** The method for producing an alkane according to claim 1 or 2,
wherein the step of reacting the C8-C24 alcohol with hydrogen is performed by using the noble metal of Groups 8 to 10 of the periodic table in an amount of 0.1 to 1% by mass relative to the mass of the C8-C24 alcohol.

**4.** The method for producing an alkane according to claim 1 or 2,
wherein the reaction temperature is 180°C or higher.

**5.** The method for producing an alkane according to claim 1 or 2,
wherein the hydrogen pressure is 1.0 MPa (gauge) or lower.

**6.** The method for producing an alkane according to claim 1 or 2,
wherein the hydrogen pressure is 0 MPa (gauge) or higher.

**7.** The method for producing an alkane according to claim 1 or 2,
wherein the step of reacting the C8-C24 alcohol with hydrogen has a reaction time of two to eight hours.

**8.** The method for producing an alkane according to claim 1 or 2,
wherein the zeolite catalyst has a molar ratio ($SiO_2/Al_2O_3$) of silica ($SiO_2$) to alumina ($Al_2O_3$) of 24 to 30.

**9.** The method for producing an alkane according to claim 1 or 2,
wherein the noble metal of Groups 8 to 10 of the periodic table is at least one selected from the group consisting of palladium, ruthenium, platinum, and rhodium.

**10.** An alkane having a natural origin index of 100% and a branched alkane content of 5% by mass or more and 70% by mass or less of the total alkane.

**INTERNATIONAL SEARCH REPORT**

International application No. **PCT/JP2024/034888**

**A. CLASSIFICATION OF SUBJECT MATTER**

***C07C 1/22***(2006.01)i; ***B01J 23/42***(2006.01)i; ***B01J 23/44***(2006.01)i; ***B01J 23/46***(2006.01)i; ***B01J 29/18***(2006.01)i; ***B01J 29/40***(2006.01)i; ***B01J 29/44***(2006.01)i; ***B01J 29/74***(2006.01)i; ***C07B 61/00***(2006.01)i; ***C07C 1/24***(2006.01)i; ***C07C 9/15***(2006.01)i; ***C07C 9/16***(2006.01)i; ***C07C 9/22***(2006.01)i

FI: C07C1/22; B01J23/42 Z; B01J23/44 Z; B01J23/46 301Z; B01J23/46 311Z; B01J29/18 Z; B01J29/40 Z; B01J29/44 Z; B01J29/74 Z; C07B61/00 300; C07C1/24; C07C9/15; C07C9/16; C07C9/22

According to International Patent Classification (IPC) or to both national classification and IPC

**B. FIELDS SEARCHED**

Minimum documentation searched (classification system followed by classification symbols)

C07C1/00; B01J23/00; B01J29/00; C07C9/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2015-221770 A (COSMOS TECHNICAL CENTER CO., LTD.) 10 December 2015 (2015-12-10) claims 1-5 | 10 |
| X | JP 2009-539944 A (NESTE OIL OYJ) 19 November 2009 (2009-11-19) claims 1-16 | 10 |
| X | JP 2009-518533 A (NESTE OIL OYJ) 07 May 2009 (2009-05-07) claims 1-17 | 10 |
| X | JP 2010-529274 A (NESTE OIL OYJ) 26 August 2010 (2010-08-26) claims 1-10 | 10 |
| X | JP 2021-508724 A (NESTE OYJ) 11 March 2021 (2021-03-11) claims 1-15 | 10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 December 2024** | **07 January 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/034888**

**C. DOCUMENTS CONSIDERED TO BE RELEVANT**

| Category* | Citation of document, with indication, where appropriate, of the relevant passages | Relevant to claim No. |
|---|---|---|
| X | JP 2020-524147 A (NESTE OYJ ) 13 August 2020 (2020-08-13)<br>claims 1-30 | 10 |
| X | WO 2017/187189 A1 (SCG CHEMICALS CO., LTD.) 02 November 2017 (2017-11-02)<br>claims 1-22, 29-30, tables 1-6 | 1-9 |
| X | US 2016/0168473 A1 (EAST CHINA NIVERSITY OF SCIENCE AND TECHNOLOGY) 16 June 2016 (2016-06-16)<br>claims 1, 15, paragraph [0011], example 4 | 1-9 |

Form PCT/ISA/210 (second sheet) (July 2022)

**INTERNATIONAL SEARCH REPORT**
**Information on patent family members**

International application No.
**PCT/JP2024/034888**

| Patent document cited in search report | Publication date (day/month/year) | Patent family member(s) | Publication date (day/month/year) |
|---|---|---|---|
| JP 2015-221770 A | 10 December 2015 | (Family: none) | |
| JP 2009-539944 A | 19 November 2009 | US 2007/0299291 A1 claims 1-20 | |
| | | WO 2007/144473 A1 | |
| | | EP 2038375 A1 | |
| | | CN 101484552 A | |
| | | KR 10-2009-0025336 A | |
| JP 2009-518533 A | 07 May 2009 | US 2007/0135669 A1 claims 1-20 | |
| | | WO 2007/068798 A2 | |
| | | EP 1795576 A1 | |
| | | CN 101331210 A | |
| | | KR 10-2008-0078889 A | |
| JP 2010-529274 A | 26 August 2010 | WO 2008/152199 A1 claims 1-10 | |
| | | EP 2155838 A1 | |
| | | CN 101679876 A | |
| | | KR 10-2010-0036307 A | |
| JP 2021-508724 A | 11 March 2021 | US 2021/0062015 A1 claims 1-20 | |
| | | WO 2019/129625 A1 | |
| | | EP 3732161 A | |
| | | CN 111527061 A | |
| JP 2020-524147 A | 13 August 2020 | US 2020/0181503 A1 claims 1-44 | |
| | | WO 2018/234190 A1 | |
| | | EP 3642179 A1 | |
| | | CN 110709375 A | |
| | | KR 10-2020-0019898 A | |
| WO 2017/187189 A1 | 02 November 2017 | EP 3448961 A1 | |
| US 2016/0168473 A1 | 16 June 2016 | WO 2015/010266 A1 | |
| | | CN 105378036 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2009519264 T